# EUROPEAN PATENT APPLICATION

(11) **EP 4 019 644 A1**
(43) Date of publication of application: **29.06.2022**
(21) Application number: 21215484.3
(22) Date of filing: 17.12.2021
(51) Int. Cl.: C12Q 1/6806

(54) **MOLECULAR DIAGNOSTIC METHOD USING CELL LYSIS COMPOSITION FOR NUCLEIC ACID EXTRACTION**

(30) Priority: 28.12.2020 KR 20200184288
(71) Applicant: LG Chem, Ltd., Seoul 07336 (KR)
(72) Inventor: Kim, Se Ryun, 34122 Yuseong-gu, Daejeon (KR); Park, Chang Ju, 34122 Yuseong-gu, Daejeon (KR); Kim, Jan Di, 34122 Yuseong-gu, Daejeon (KR); Park, So Hyun, 34122 Yuseong-gu, Daejeon (KR); Oh, Jae Hoon, 34122 Yuseong-gu, Daejeon (KR)
(74) Representative: Hoffmann Eitle

(57) **Abstract**

The present invention relates to a molecular diagnostic method using a cell lysis composition for nucleic acid extraction, and particularly, to a molecular diagnostic method using a cell lysis composition for nucleic acid extraction, which may minimize the time for molecular diagnosis by performing a polymerase chain reaction on a mixture containing both Tween 20 as a component for nucleic acid extraction and a PCR buffer necessary for performing the polymerase chain reaction, without a separate nucleic acid extraction process, and reduce the cost of molecular diagnosis by minimizing the use of dedicated devices and consumables in extraction.

## Description

### BACKGROUND

### 1. Technical Field

The Present invention claims the benefit of the filing date of Korean Patent Application No. 10-2020-0184288, filed on December 28, 2020, in the Korean Intellectual Property Office, the entire content of which is included in the present invention.
The present invention relates to a molecular diagnostic method using a cell lysis composition for nucleic acid extraction, and particularly, to a molecular diagnostic method using a cell lysis composition for nucleic acid extraction, which may minimize the time for molecular diagnosis by performing a polymerase chain reaction on a mixture containing both Tween 20 as a component for nucleic acid extraction and a PCR buffer necessary for performing the polymerase chain reaction, without a separate nucleic acid extraction process, and reduce the cost of molecular diagnosis by minimizing the use of dedicated devices and consumables in extraction.

### 2. Related Art

As the causes of diseases have been analyzed at the genetic level based on the results of recent human genome research, there has been a gradually increasing demand for manipulation and biochemical analysis of biological samples for the purpose of treating or preventing human diseases. In addition, there has been a demand for a technique for extracting and analyzing a nucleic acid from a biological sample or a cell-containing sample in various fields such as new drug development, pre-test for viral or bacterial infection, and forensic medicine, in addition to disease diagnosis.

Meanwhile, for molecular diagnosis, it is common to extract a nucleic acid, which is DNA or RNA containing genetic information, from saliva or blood of a person infected with a virus or bacterium, amplify the extracted nucleic acid, and confirm whether or not the person has been infected with the disease.

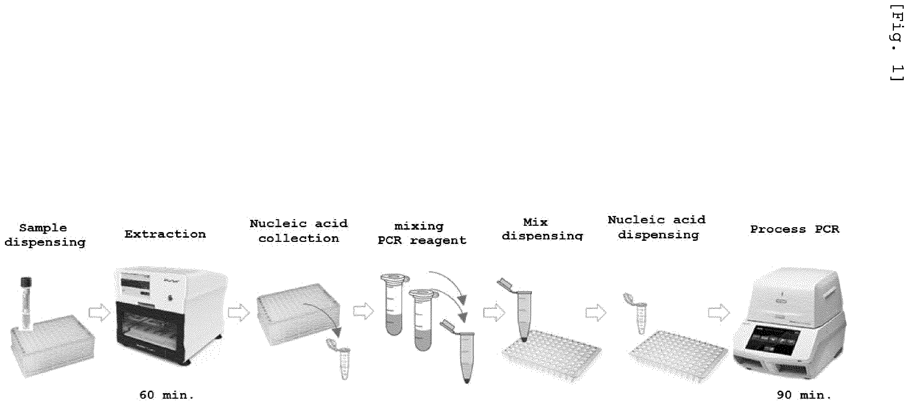

FIG. 1 is a schematic diagram showing a polymerase chain reaction according to a conventional art. In the conventional art, in order to extract a nucleic acid containing genetic information from a sample, sample dispensing is performed and then a nucleic acid is extracted from the sample. The process of extracting the nucleic acid takes about 60 minutes. Next, the nucleic acid is collected, and PCR reagents are mixed and dispensed, and then PCR is performed. It is common to amplify the nucleic acid by PCR and analyze the result of nucleic acid amplification. In order to extract the nucleic acid, lysis, purification and elution processes are required. However, in the lysis, purification and elution processes that are performed for nucleic acid extraction, dedicated extraction devices for performing these processes and articles consumed for extraction (tools made of plastic, magnetic beads or a solution) are required.

When a nucleic acid is extracted using the above-described conventional art, the nucleic acid may be extracted with high purity, but a problem arises in that the extraction process requires a long period, and thus the conventional art is not suitable for diagnosis or medical examination in emergency situations or emergency rooms. In addition, when the conventional art is applied to a situation where a national quarantine against virus is required due to the rapid spread of the virus, it is necessary to continuously use dedicated devices and consumables for extraction, and thus a problem arises in that high costs are incurred for diagnosis.

Therefore, in order to solve the above-described problems, there is an urgent need for the development of a technology capable of simultaneously performing nucleic acid extraction and amplification in the polymerase chain reaction without a separate nucleic acid extraction process by using a specific composition.

### SUMMARY

An object of the present invention is to provide a molecular diagnostic method using a cell lysis composition for nucleic acid extraction, which may omit a separate nucleic acid extraction process by performing a polymerase chain reaction on a mixture containing both a cell lysis composition for extracting a nucleic acid from cells and a solution necessary for the polymerase chain reaction, and achieve nucleic acid extraction and amplification in the polymerase chain reaction.

However, objects of the present invention are not limited to the above-mentioned object, and other objects that are not mentioned herein will be clearly understood by those skilled in the art from the following description.

One embodiment of the present invention provides a molecular diagnostic method including steps of: preparing a mixture containing: a cell lysis composition for nucleic acid extraction containing Tween 20 and distilled water; a nucleic acid-containing sample; a premix; and a solution containing primers and a probe; and extracting and amplifying the nucleic acid by performing a polymerase chain reaction on the mixture.

### BRIEF DESCRIPTION OF THE DRAWINGS

FIG. 1 is a schematic view showing a polymerase chain reaction according to a conventional art.
FIG. 2 is a flow chart showing a molecular diagnostic method using a cell lysis composition for nucleic acid extraction according to one embodiment of the present invention.
FIG. 3 is a graph showing fluorescence values as a function of the cycle number in polymerase chain reactions according to Experimental Examples 1 to 6.

### DETAILED DESCRIPTION

Throughout the present specification, it is to be understood that when any part is referred to as "including" or "containing" any component, it does not exclude other components, but may further include other components, unless otherwise specified.

Hereinafter, the present invention will be described in more detail.

One embodiment of the present invention provides a molecular diagnostic method including steps of: preparing a mixture containing: a cell lysis composition for nucleic acid extraction containing Tween 20 and distilled water; a nucleic acid-containing sample; a premix; and a solution containing primers and a probe; and extracting and amplifying the nucleic acid by performing a polymerase chain reaction on the mixture.

The molecular diagnostic method using a cell lysis composition for nucleic acid extraction according to one embodiment of the present invention may minimize the time required for a molecular diagnosis process based on nucleic acid amplification by omitting a separate nucleic acid extraction process and performing a polymerase chain reaction on the above-described mixture, and reduce the cost of molecular diagnosis by minimizing the use of dedicated devices and consumables in extraction.

Referring to FIG. 1, in a molecular diagnostic method according to a conventional art, lysis, purification and elution processes are performed to extract a nucleic acid such as DNA or RNA from cells, and an additional PCR buffer is added to the eluted solution, followed by a polymerase chain reaction (PCR). Then, the nucleic acid amplified by the PCR is generally used for diagnosis. However, the above method has problems in that dedicated devices for the lysis, purification and elution processes are required, and in that various consumables such as solutions or plastic wires should be continuously used for these processes.

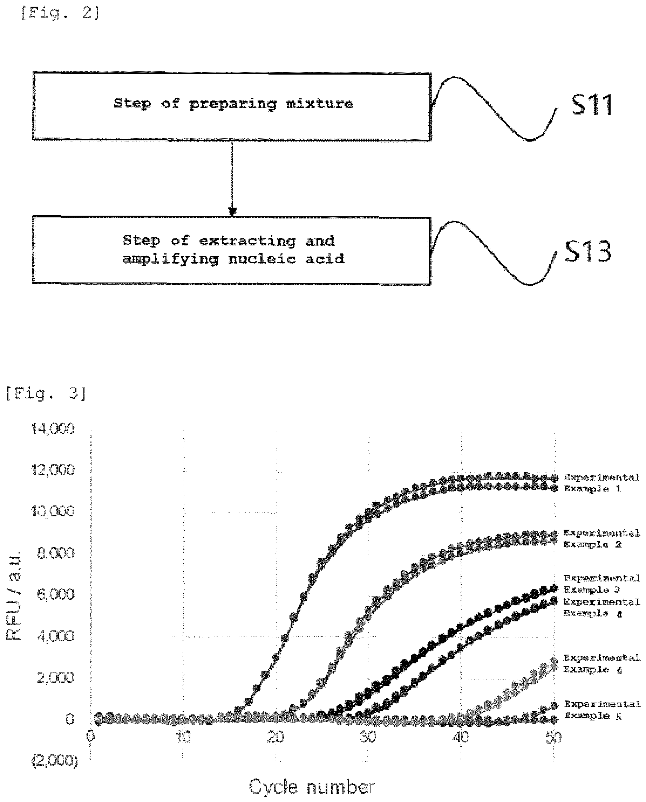

FIG. 2 is a flow chart showing a molecular diagnostic method using a cell lysis composition for nucleic acid extraction according to one embodiment of the present invention. Referring to FIG. 2, according to the present invention, it is possible to simultaneously achieve nucleic acid extraction and amplification using a simple method by performing a step (S11) of preparing a mixture containing a cell lysis composition, a nucleic acid-containing sample, a premix, and a solution containing primers and a probe, and then performing a step (S13) of extracting and amplifying the nucleic acid by performing a polymerase chain reaction on the mixture. Specifically, according to the present invention, a solution that is used in the cell wall lysis process is limited to a specific type of solution, and reagents that are generally used in the conventional art are not added to the solution. However, a cell lysis composition containing only Tween 20 as a component for nucleic acid extraction is used, and a PCR buffer for performing PCR, that is, a premix, primers and a probe are additionally added to the cell lysis composition, and PCR is performed using the resulting mixture. Thus, it is possible to extract and amplify the nucleic acid by performing PCR without a separate nucleic acid extraction process and perform molecular diagnosis.

According to one embodiment of the present invention, the cell lysis composition for nucleic acid extraction may be a mixture containing only Tween 20 and distilled water. More specifically, the cell lysis composition for nucleic acid extraction may consist of Tween 20 (polysorbate 20) and distilled water. Where the components of the cell lysis composition for nucleic acid extraction are controlled as described above, it is possible to simplify molecular diagnosis and shorten the time required for molecular diagnosis by omitting a nucleic acid extraction process and simultaneously achieving nucleic acid extraction and amplification in PCR.

According to one embodiment of the present invention, the molecular diagnostic method includes a step of preparing a mixture containing: a cell lysis composition for nucleic acid extraction containing Tween 20 and distilled water; a nucleic acid-containing sample; a premix; and a solution containing primers and a probe. Specifically, the cell lysis composition for nucleic acid extraction may contain only Tween 20 and distilled water, and the premix and the solution containing the primers and the probe may be PCR buffers. As the molecular diagnostic method includes the step of preparing the mixture as described above, it is possible to simultaneously achieve nucleic acid extraction and amplification by performing PCR without a separate nucleic acid extraction process, thereby reducing the time required for molecular diagnosis.

According to one embodiment of the present invention, the molecular diagnostic method includes a step of extracting and amplifying the nucleic acid by performing a polymerase chain reaction on the mixture. As the nucleic acid is extracted and amplified by performing a polymerase chain reaction on the mixture, it is possible to ensure a nucleic acid for molecular diagnosis and minimize the time required for molecular diagnosis.

According to one embodiment of the present invention, the molecular diagnostic method does not include a separate step of extracting the nucleic acid from the nucleic acid-containing sample. As the molecular diagnostic method does not include the separate step of extracting the nucleic acid, it is possible to reduce the time required for molecular diagnosis and reduce the cost because dedicated devices or consumables for nucleic acid extraction are not used.

According to one embodiment of the present invention, the concentration of Tween 20 in the cell lysis composition may be 2.0 vol/vol% or more and less than 16.0 vol/vol%. Specifically, the concentration of Tween 20 may be 2.2 vol/vol% to 15.8 vol/vol%, 2.4 vol/vol% to 15.6 vol/vol%, 2.6 vol/vol% to 15.4 vol/vol%, 2.8 vol/vol% to 15.2 vol/vol%, 3.0 vol/vol% to 15.0 vol/vol%, 3.2 vol/vol% to 14.8 vol/vol%, 3.4 vol/vol% to 14.6 vol/vol%, 3.6 vol/vol% to 14.4 vol/vol%, 3.8 vol/vol% to 14.2 vol/vol%, 4.0 vol/vol% to 14.0 vol/vol%, 4.2 vol/vol% to 13.8 vol/vol%, 6.0 vol/vol% to 12.0 vol/vol%, 7.0 vol/vol% to 11.0 vol/vol%, 8.0 vol/vol% to 10.0 vol/vol%, or 8.0 vol/vol% to 9.0 vol/vol%. By adjusting the concentration of Tween 20 within the above-described range, it is possible to improve the effect of lysing the cells, and it is possible to shorten the time required for molecular diagnosis by extracting and amplifying the nucleic acid by PCR without a separate additional process.

According to one embodiment of the present invention, as a sample for nucleic acid analysis, that is, a nucleic acid-containing sample, is added to the cell lysis composition for nucleic acid extraction, the concentration of a detergent in the mixture containing the sample may change. Specifically, as the cell lysis composition for nucleic acid extraction and the sample are mixed in the same volumes, the concentration of the detergent in the mixture containing the sample may decrease to half of the concentration of the above-described cell lysis composition for nucleic acid extraction. Throughout the present specification, the term "nucleic acid-containing sample" may refer to a sample containing a nucleic acid in a sample, such as a sample containing a DNA or RNA nucleic acid in a cell, or a sample containing an eluted nucleic acid in a cell.

According to one embodiment of the present invention, the volume ratio between the sample and the cell lysis composition may be 1:0.5 to 1:2.0. Where the volume ratio between the sample and the cell lysis composition is controlled within the above-described range, it is possible to extract the nucleic acid from the cells within a short time by controlling the concentration of Tween 20.

According to one embodiment of the present invention, the volume ratio between the premix and the solution containing primers and a probe may be 1:1 to 1:10. Where the volume ratio between the premix and the solution containing primers and a probe is controlled within the above-described range, it is possible to reduce the time required for molecular diagnosis by maximizing the effect of extracting and amplifying the nucleic acid.

According to one embodiment of the present invention, the volume ratio between the mixture containing the nucleic acid extract; and the premix may be 1:0.5 to 1:2.0. Where the volume ratio between the mixture containing the nucleic acid extract; and the premix is controlled within the above-described range, it is possible to reduce the time required for molecular diagnosis by maximizing the effect of extracting and amplifying the nucleic acid.

According to one embodiment of the present invention, the step of extracting and amplifying the nucleic acid may be performed at a temperature of higher than 60°C and not higher than 100°C. Specifically, the step of extracting and amplifying the nucleic acid may be performed at a temperature of 61°C to 99°C, 62°C to 98°C, 63°C to 97°C, 64°C to 96°C, 65°C to 95°C, 66°C to 94°C, 67°C to 93°C, 68°C to 92°C, 69°C to 91°C, or 70°C to 90°C. More specifically, the step of extracting and amplifying the nucleic acid is preferably performed at a temperature of 50°C to 100°C. Where the temperature of the step of extracting and amplifying the nucleic acid is controlled within the above-described range, it is possible to improve the effect of lysing the cells and reduce the time required for molecular diagnosis by amplifying the nucleic acid by PCR without a separate nucleic acid extraction process.

According to one embodiment of the present invention, the molecular diagnostic method may further include a step of keeping the mixture in an incubator, after the step of preparing the mixture. Where the mixture is kept in the incubator after the step of preparing the mixture as described above, it is possible to improve the effect of extracting the nucleic acid. As used in the present specification, the term "incubator" refers to a device in which a space is formed and whose temperature, humidity and pressure may be controlled. This device is not limited to the above name.

According to one embodiment of the present invention, the step of keeping the mixture in the incubator may be performed for 1 minute to 10 minutes. Where the time during which the mixture is kept in the incubator is controlled within the above-described range, it is possible to improve the effect of lysing the cells.

According to one embodiment of the present invention, the molecular diagnostic method may further include a step of analyzing the solution containing the extracted and amplified nucleic acid, after the step of extracting and amplifying the nucleic acid by performing the polymerase chain reaction on the mixture. Where the step of analyzing the solution containing the extracted and amplified nucleic acid is further included as described above, it is possible to minimize the time required for molecular diagnosis based on nucleic acid amplification and reduce the cost of molecular diagnosis by minimizing the use of dedicated devices and consumables in nucleic acid extraction.

Hereinafter, the present invention will be described in detail with reference to examples. However, the examples according to the present invention may be modified into various different forms, and the scope of the present invention is not interpreted as being limited to the examples described below. The examples of the present specification are provided to more completely explain the present invention to those skilled in the art.

### Experimental Example 1

In order to amplify a 16S rRNA which is the target gene of *E coli* DH3 as a test object, the gene was extracted by a spin-column extraction method. Specifically, after the test object *E coli* DH3 was collected, the 16S rRNA was extracted from the test object by performing a lysis process, a purification process and an elution process using a conventional Qiagen extraction method.

Then, an intercalating dye (Ssofast pol. EvaGreen, Green) was added to the mixture containing the extract, and a preliminary reaction was performed at 95°C for 2 minutes. Then, the target gene 16S rRNA was amplified by performing PCR for 50 cycles, each consisting of 95°C for 10 seconds and then 61°C for 10 seconds. Fluorescence values were measured for each cycle, and the threshold cycle (Ct) value that is the minimum threshold value capable of determining the result of nucleic acid amplification was measured in this process. The results of the measurement are summarized in Table 1 below.

### Experimental Example 2

In order to amplify a 16S rRNA which is the target gene of *E coli* DH3 as a test object, the gene was extracted by an E3 extraction method. More specifically, the E3 extraction method is an automated nucleic acid extraction method, and may be roughly divided into an extraction reagent (E3 nucleic acid (NA) extraction kit) and an extraction system. The E3 extraction method is a method of extracting a nucleic acid (DNA/RNA) from a human sample using a magnetic bead coated with iron oxide. The reagent tube has four wells and contains a lysis buffer, washing buffer and elution buffer. High-concentration chaotropic salt contained in the lysis buffer and washing buffer serves to destroy cell membranes and separate nucleic acids from proteins, and in this environment, a negatively charged nucleic acid is adsorbed on a magnetic bead. The nucleic acid adsorbed on the magnetic bead is sequentially moved to each column of the tube by a magnetic rod of the nucleic acid extraction device (E3 system) and undergoes several washing steps. Finally, the elution buffer separates the nucleic acid adsorbed on the magnetic bead, and the magnetic rod removes only the magnetic bead, leaving only a pure nucleic acid in the elution buffer. The 16S rRNA was extracted by the above-described method.

Next, an intercalating dye (Ssofast pol. EvaGreen, Green) was added to the mixture containing the extract, and a preliminary reaction was performed at 95°C for 2 minutes. Then, the target gene 16S rRNA was amplified by performing PCR for 50 cycles, each consisting of 95°C for 10 seconds and then 61°C for 10 seconds. Fluorescence values were measured for each cycle, and the threshold cycle (Ct) value that is the minimum threshold value capable of determining the result of nucleic acid amplification was measured in this process. In addition, ΔCt which is the difference of the Ct of Experimental Example 2 from the Ct of Experimental Example 1 was calculated. The results are summarized in Table 1 below.

### Experimental Example 3

In order to amplify a 16S rRNA which is the target gene of *E coli* DH3 as a test object, a cell lysis composition for nucleic acid extraction containing Tween 20 and distilled water was prepared to have a Tween 20 concentration of 10 vol/vol%, and a sample containing the test object was added to the composition, thus preparing a mixture having a Tween 20 concentration controlled to 5 vol/vol%. Thereafter, the mixture was kept in an incubator at room temperature for 5 minutes, and then centrifuged using a centrifuge at 15,000 g for 15 minutes.

Next, an intercalating dye (Ssofast pol. EvaGreen, Green) was added to the mixture containing the extract, and a preliminary reaction was performed at 95°C for 2 minutes. Then, the target gene 16S rRNA was amplified by performing PCR for 50 cycles, each consisting of 95°C for 10 seconds and then 61°C for 10 seconds. Fluorescence values were measured for each cycle, and the threshold cycle (Ct) value that is the minimum threshold value capable of determining the result of nucleic acid amplification was measured in this process. In addition, ΔCt which is the difference of the Ct of Experimental Example 3 from the Ct of Experimental Example 1 was calculated. The results are summarized in Table 1 below.

### Experimental Example 4

The fluorescence value for each cycle and the Ct (threshold cycle) value were measured in the same manner as in Experimental Example 3, except that the Tween 20 concentration in the cell lysis composition for nucleic acid extraction was controlled to 20 vol/vol% and the Tween 20 concentration in the mixture was controlled to 10 vol/vol%. In addition, ΔCt which is the difference of the Ct of Experimental Example 4 from the Ct of Experimental Example 1 was calculated. The results are summarized in Table 1 below.

### Experimental Example 5

The fluorescence value for each cycle and the Ct (threshold cycle) value were measured in the same manner as in Experimental Example 3, except that the cell lysis composition for nucleic acid extraction contained Triton X-100 instead of Tween 20. In addition, ΔCt which is the difference of the Ct of Experimental Example 5 from the Ct of Experimental Example 1 was calculated. The results are summarized in Table 1 below.

### Experimental Example 6

The fluorescence value for each cycle and the Ct (threshold cycle) value were measured in the same manner as in Experimental Example 4, except that the cell lysis composition for nucleic acid extraction contained Triton X-100 instead of Tween 20. In addition, ΔCt which is the difference of the Ct of Experimental Example 6 from the Ct of Experimental Example 1 was calculated. The results are summarized in Table 1 below.

**[Table 1]**

| | Ct (number) | ΔCt (number) |
|---|---|---|
| Experimental Example 1 | 17.5 | - |
| Experimental Example 2 | 23.5 | 6.0 |
| Experimental Example 3 | 29.2 | 11.7 |
| Experimental Example 4 | 32.8 | 15.3 |
| Experimental Example 5 | 50.0 or more | 32.5 or more |
| Experimental Example 6 | 44.4 | 26.9 |

FIG. 3 is a graph showing fluorescence values as a function of the cycle number in polymerase chain reactions according to Experimental Examples 1 to 6. Referring to FIG. 3 and Table 1 above, it was confirmed that the fluorescence value and Ct value in Experimental Example 3, in which the cell lysis composition for nucleic acid extraction containing Tween 20 at a concentration of 10 vol/vol% was used, were similar to those in Experimental Examples 1 and 2 corresponding to a conventional nucleic acid extraction method. However, it was confirmed that, since a purification process and an elution process were not performed in the nucleic acid extraction process of Experimental Example 3, the time required for molecular diagnosis was shorter in Experimental Example 3 than in Experimental Examples 1 and 2.

In contrast, it was confirmed that, in Experimental Examples 5 and 6 in which the cell lysis composition for nucleic acid extraction containing Triton X-100 was used, the Ct value increased at the same fluorescence value (RFU) because the cell wall lysis within the same time was delayed.

Furthermore, it was confirmed that, in Experimental Example 4 in which Tween 20 was used at a concentration of 20 vol/vol%, the Ct value increased compared to that in Experimental Example 3, in which Tween 20 was used at a concentration of 10 vol/vol%, in order to have the same fluorescence value (RFU).

Through Experimental Examples 1 to 3, it was confirmed that Tween 20 is suitable as a component of a cell lysis composition for extracting a nucleic acid from cells.

In the following Examples, the effect of the molecular diagnostic method according to one embodiment of the present invention was compared with the effect of a conventional molecular diagnostic method.

### Comparative Example 1

In order to amplify the gene contained in each of *M. tuberculosis, M. avium and M. intracellulare* as test objects, the gene was extracted by a spin-column extraction method. Specifically, the test objects *M. tuberculosis, M. avium* and *M. intracellulare* were collected, and then the gene was extracted from each of the test objects by performing all of a lysis process, a purification process and an elution process using a conventional Qiagen extraction method.

Next, 4 µl of a primer/probe mixture capable of specifically detecting *M. tuberculosis* and nontuberculous mycobacteria (NTM) (including *M. avium* and *M. intracellulare)* and 20 µl of Taq polymerase premix (Realhelix^{™} qPCR Kit, NanoHelix, Korea) were added to 16 µl of the extract-containing mixture, and then a preliminary reaction was performed at 95°C for 5 minutes. Thereafter, the gene was amplified by performing PCR for 40 cycles, each consisting of 95°C for 10 seconds and then 60°C for 40 seconds. The threshold cycle (Ct) value that is the minimum threshold value capable of determining the result of gene amplification was measured in this process. The results of the measurement are summarized in Table 2 below.

### Comparative Example 2

In order to amplify the gene contained in each of *M. tuberculosis, M. avium and M. intracellulare* as test objects, a cell lysis composition for nucleic acid extraction was prepared by mixing a measured volume of Tween 20 with a measured volume of distilled water so that the concentration of Tween 20 in the composition was 5.0 vol/vol%. Then, 20 µl of a cell-containing sample was mixed with 20 µl of the cell lysis composition for nucleic acid extraction, thereby preparing a sample-containing mixture in which the concentration of Tween 20 as a detergent was 2.5 vol/vol%. Then, a nucleic acid was extracted from the cells by lysing the cells at 90°C for 5 minutes.

Next, 4 µl of a primer/probe mixture capable of specifically detecting *M. tuberculosis* and nontuberculous mycobacteria (NTM) (including *M. avium* and *M. intracellulare)* and 20 µl of Taq polymerase premix (Realhelix^{™} qPCR Kit, NanoHelix, Korea) were added to 16 µl of the extract-containing mixture, and then a preliminary reaction was performed at 95°C for 5 minutes. Then, the target gene IS6110 and ITS region were amplified by performing PCR for 40 cycles, each consisting of 95°C for 10 seconds and then 60°C for 40 seconds. Fluorescence values were measured for each cycle, and the threshold cycle (Ct) value that is the minimum threshold value capable of determining the result of nucleic acid amplification was measured in this process. In addition, ΔCt which is the difference of the Ct of Comparative Example 2 from the Ct of Comparative Example 1 was calculated. The results are summarized in Table 2 below.

### Example 1

In order to amplify the gene contained in each of *M. tuberculosis, M. avium* and *M. intracellulare* as test objects, a cell lysis composition for nucleic acid extraction was prepared by mixing a measured volume of Tween 20 with a measured volume of distilled water so that the concentration of Tween 20 in the composition was 12.5 vol/vol%. Then, 20 µl of a cell-containing sample was mixed with 20 µl of the cell lysis composition for nucleic acid extraction, thereby preparing a sample-containing mixture in which the concentration of Tween 20 as a detergent was 6.25 vol/vol% (then, the concentration of Tween 20 was controlled so that the final concentration of Tween 20 in 40 µl of the total volume of the following PCR reaction solution was 2.5 vol/vol%).

Next, 20 µl of Taq polymerase premix (Realhelix^{™} qPCR Kit, NanoHelix, Korea) and 4 µl of a primer/probe-containing solution capable of specifically detecting *M. tuberculosis* and nontuberculous mycobacteria (NTM) (including *M. avium* and *M. intracellulare)* were added to 16 µl of the mixture, and then a preliminary reaction was performed at 95°C for 5 minutes. Then, the gene was amplified by performing PCR for 40 cycles, each consisting of 95°C for 10 seconds and then 60°C for 40 seconds. The threshold cycle (Ct) value that is the minimum threshold value capable of determining the result of nucleic acid amplification was measured in this process. The results are summarized in Table 2 below.

**[Table 2]**

| Test object | Comparative Example 1 [unit: | Comparative Example 2 [unit: | Example 1 [unit: Ct] |
|---|---|---|---|
| | Ct] | Ct] | |
| *M. tuberculosis* | 32.13 | 33.89 | 32.53 |
| *M. avium* | 32.83 | 32.02 | 30.44 |
| *M. intracellulare* | 29.98 | 28.87 | 30.40 |

Furthermore, for each test object shown in Table 2 above, ΔCt which is the difference in Ct between Comparative Example 1, Comparative Example 2 and Example 1 was calculated, and the results are summarized in Table 3 below.

**[Table 3]**

| Test object | ΔCt (Comparative Example 1-Comparative Example 2) [unit: Ct] | ΔCt (Comparative Example 2-Example 1) [unit: Ct] | ΔCt (Comparative Example 1-Example 1) [unit: Ct] |
|---|---|---|---|
| *M. tuberculosis* | -1.76 | 1.36 | -0.40 |
| *M. avium* | 0.81 | 1.58 | 2.39 |
| *M. intracellulare* | 1.11 | -1.53 | -0.42 |
| Average | 0.05 | 0.47 | 0.52 |

Referring to Tables 2 and 3 above, it was confirmed that there was an average Ct difference of 0.52 between Comparative Example 1, in which the nucleic acid was extracted by the conventional Qiagen spin-column extraction method and amplified by performing PCR, and Example 1 which is one embodiment of the present invention. In addition, it was confirmed that there was an average Ct difference of 0.47 between Comparative Example 2, in which the nucleic acid was extracted using the cell lysis composition containing only Tween 20, and then amplified by performing PCR, and Example 1 which is one embodiment of the present invention.

In conclusion, it was confirmed that Example 1 according to one embodiment of the present invention exhibited a Ct value similar to or lower than those of Comparative Examples 1 and 2 corresponding to a conventional art, but the time required for molecular diagnosis was reduced in Example 1 compared to the convention art because there was no nucleic acid extraction process in Example 1. In addition, it was confirmed that the cost required for molecular diagnosis was reduced in Example 1 because consumables were not used in Example 1.

Therefore, the molecular diagnostic method using a cell lysis composition for nucleic acid extraction according to one embodiment of the present invention may omit a separate nucleic acid extraction process and achieve nucleic acid extraction and amplification in a polymerase chain reaction, by performing the polymerase chain reaction on a mixture containing both Tween 20 as a component for nucleic acid extraction and a PCR buffer necessary for performing the polymerase chain reaction.

As described above, the molecular diagnostic method using a cell lysis composition for nucleic acid extraction according to one embodiment of the present invention may minimize the time for a molecular diagnostic process based on nucleic acid amplification by omitting a separate nucleic acid extraction process and performing a polymerase chain reaction on a mixture containing the cell lysis composition, and reduce the cost of molecular diagnosis by minimizing the use of dedicated devices and consumables in nucleic acid extraction.

The effects of the present invention are not limited to the above-mentioned effects, and effects which are not mentioned herein will be clearly understood by those skilled in the art from the disclosures in the specification and the accompanying drawings.

Although the present invention has been described above with reference to limited embodiments, it should be understood that the present invention is not limited to these embodiments, and various modifications and variations may be made by those skilled in the art without departing from the technical idea of the present invention and within the range of equivalents to the following claims.

## Claims

1. A molecular diagnosis method comprising:
preparing a mixture containing: a cell lysis composition for nucleic acid extraction containing Tween 20 and distilled water; a nucleic acid-containing sample; a premix; and a solution containing primers and a probe; and
extracting and amplifying a nucleic acid of the nucleic acid-containing sample by performing a polymerase chain reaction on the mixture.

2. The molecular diagnosis method of claim 1, wherein a concentration of Tween 20 in the composition is 2.0 vol/vol% or more and less than 16.0 vol/vol%.

3. The molecular diagnosis method of claim 1, wherein a volume ratio between the sample and the cell lysis composition is 1:0.5 to 1:2.0.

4. The molecular diagnosis method of claim 1, wherein a volume ratio between the premix and the solution containing the primers and the probe is 1:1 to 1:10.

5. The molecular diagnosis method of claim 1, wherein a volume ratio between a mixture containing the extracted nucleic acid; and the premix is 1:0.5 to 1:2.0.

6. The molecular diagnosis method of claim 1, wherein the extracting and amplifying the nucleic acid is performed at a temperature of higher than 60°C and not higher than 100°C.

7. The molecular diagnosis method of claim 1, further comprising keeping the mixture in an incubator, after the preparing the mixture.

8. The molecular diagnosis method of claim 7, wherein the keeping the mixture in the incubator is performed for 1 minute to 10 minutes.
